# EUROPEAN PATENT SPECIFICATION

(11) **EP 2 937 046 B1**
(45) Date of publication and mention of the grant of the patent: **06.03.2019**
(21) Application number: 12890611.2
(22) Date of filing: 19.12.2012
(51) Int. Cl.: A61B 17/17, A61B 17/80

(54) **PLATE-CENTERING DEVICE FOR ORTHOPEDIC SURGERY**
PLATTENZENTRIERUNGSVORRICHTUNG FÜR ORTHOPÄDISCHE CHIRURGIE
CENTREUR DE PLAQUE POUR CHIRURGIE ORTHOPÉDIQUE

(43) Date of publication of application: 28.10.2015
(73) Proprietor: Industrias Medicas Sampedro S.A.S., Sabaneta, Antioquia (CO)
(72) Inventor: TORO, Mauricio, Sabaneta Antioquia (CO); GOMEZ, Jairo, Fernando, Sabaneta Antioquia (CO)
(74) Representative: Maldonado Jordan, Julia
(86) International application number: PCT/IB2012/057493
(87) International publication number: WO 2014/096904

(56) References cited:
- EP-A1- 1 943 967
- CN-Y- 2 708 855
- FR-A1- 2 705 885
- US-A- 5 713 898
- US-A1- 2011 106 183
- US-A1- 2012 095 466
- US-A1- 2012 303 068
- US-B2- 7 815 681

## Description

### TECHNICAL FIELD

The present invention relates to a device used for centering a plate during orthopedic surgery, especially of the distal radius, where said plate centering device is manufactured from polymeric, highly elastic material that allows it to adapt to different bone morphologies. Thus, the plate centering device is used in orthopedic surgery, particularly in those related to peri-articular fractures, in which the commonly used practice is to attach the plate starting at the epiphysiary zone. Further, it is common when attaching this zone to lose the plate's orientation with the remaining bone hence making difficult the fracture's reduction, making the surgical process more complicated.

In this manner, the plate centering device of the present invention, by being elastic it wraps around the bone at the diaphysary portion, guaranteeing that the plate does not lose its orientation while it is being attached to the epiphisary portion. This attachment can be supported, if necessary, by a clamp that increases the pressure on the bone, such as the clamps normally used by surgeons in the operating room when carrying out operation of this type, where said clamp allows for the centering device to be attached by means of a central channel covering all the outer portion of said centering device and allows both the surgeon as well as any of his assistant's during the surgery to keep the plate in place while the surgery is being carried out.

### BACKGROUND OF THE INVENTION

Currently, most orthopedic surgeries related to fractures and requiring installation of a plate for joining the fractured bone, it is carried out by means of a complex procedure which also requires a longer recovery by the patient. For this reason, it is necessary for said procedure to be carried out in a successful manner and only once, in other words, taking into consideration that both the surgery and the recovery are traumatic for the patient, it is undesirable for there to be any inconvenient with the plates or the bonding of the broken bone, so the patient can have an adequate recovery.

In this way, the plates commonly used in these type of procures require a series of screws and accessories or additional elements that must keep them in place, preventing it from moving and producing an undesired bone regeneration which could lead to malformation on the patient's bones. For this it is necessary that the plate is placed on its implant location and attached, which is commonly carried out by means of a series of clamps that let the plate to be in place while the operation is carried out, but once it is over they are removed such that only the screws keep said plate in place, which is not always desirable in every case since there can be movements of the plate and an inadequate bone regeneration.

Therefore, in the state of the art there is a plurality of disclosures related with useful devices when carrying out orthopedic surgery where it is required that the plate being installed on the patient must remain in its corresponding position and, in that way, avoid problems due to a bad placement of the plate during surgery.

In this sense, document CN 1686057 refers to an adaptation plate centering device, where said device is of the type with centered teeth for treating bone fractures, and said device is adequate for installing bone plates during surgery, where the same is comprised of an arc base plate with an arc cross section, screw holes, holes for installing pressure screws and bone regulator holes, hooked shaped angular side undulations (ribs) with a cutting tip, wherein the teeth are arranged on the cutting tip of the longitudinal angular rib at intervals, such that the device couples with the fracture site and does not move, just as it is necessary for this type of treatment or surgery.

Nonetheless, the invention described in this anteriority presents a series of teeth on the inner part allowing the device to remain in position and not move, which fact could be regarded as a disadvantage since said teeth do not assure that the plate is kept in place, since also there is no device, such as an inner guide, so there is no displacement of the plate.

On the other hand, document EP 1943967 discloses a centering device for orthopedic surgery, which is used in operations requiring intra-medullary binding, where said device is comprised of a metallic frame of a "C" shaped semi-circular section provided with an amplifier pointed towards an X-ray tube, which comprises at least one fixed bar attached to the same C-shaped frame and wherein it also comprises a series of mobile means cooperating with the incident beam emitted by the X-ray tube.

Nonetheless, the invention described in this document presents the disadvantage of requiring a series of devices for a surgery carried out by means of the use of X-ray and complex electronic systems, which is undesirable since it increases the costs associated with the procedure.

Now, document US 2011238184 is related with methods and instruments for interbody fusion, which are useful when carrying out laparoscopic surgery for preparing a site for implanting a fusion device or implant. The device mentioned in this document allows to carry out the centering of a system for laparoscopic surgery by means of a device which is inserted in the patent's bone and allows the laparoscopy screw to pass through the place desired by the surgeon.

Nonetheless, the device described in this anteriority has the disadvantage of having to be implanted directly into the patient's bone and acts as a guide for the laparoscopy device, increasing at the same time the patient's recovery due to the implanted device.

Document US 2012303068 A1 discloses a device intended to maintain temporary engagement with a bone plate to be used during surgery and a patient's bone. The device comprises a C-shaped body including two opposite ends; a central channel located on a middle portion of the C-shaped body between the two ends, with the central channel being dimensioned for positioning the bone plate; a channel located along at least a portion of an outer edge of the C-shaped body; and a fastening orifice located on an inside portion of the channel, the fastening orifice allowing the device to be attached to the patient's bone by means of a screw that goes through the fastening orifice along with the bone plate.

According to the above, it can be clearly seen that there exists in the state of the art a need to design and Implement a device to be used in orthopedic surgery, which allows to keep a bone coupling plate in its place and centering it when said procedure is being carried out, where it is required for the device to be easy to use, economic and also counts with a system to be used with the clamps commonly used by surgeons of these types of surgeries, wherein it is also desirable that the plate centering device is manufactured in a highly elastic polymeric material that allows the same to adapt to different bone morphologies, taking into consideration that every patient's bones are different.

### BRIEF DESCRIPTION OF THE FIGURES

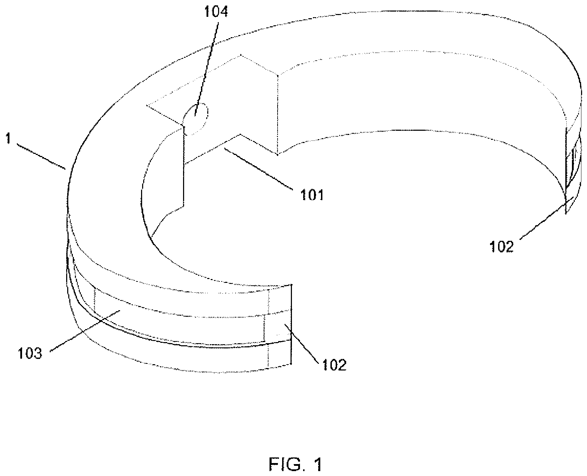

Figure 1 corresponds to a perspective view of the plate centering device of the present invention.

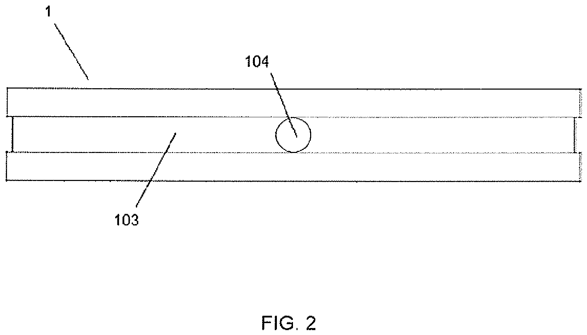

Figure 2 corresponds to a top view of the centering device of figure 1.

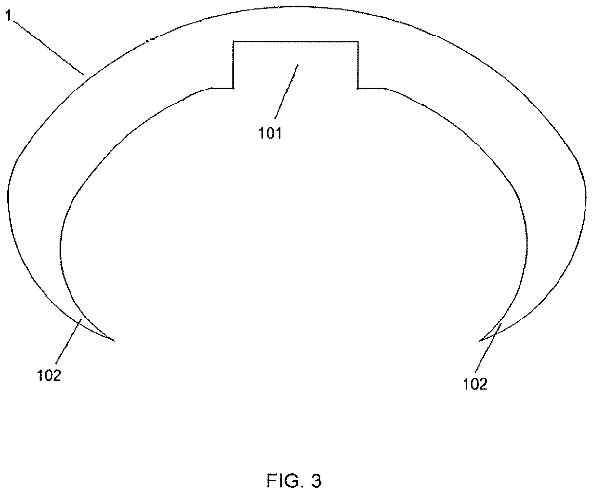

Figure 3 corresponds to a front view of the centering device of figure 1.

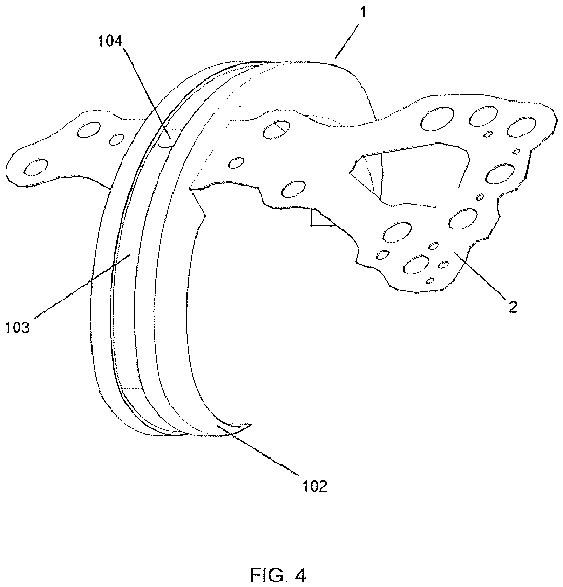

Figure 4 corresponds to a perspective view of the device of figure 1 along with a plate for orthopedic surgery.
Figure 5 corresponds to a top view of the set in figure 4 installed on a patient's bone.

### DETAILED DESCRIPTION OF THE INVENTION

The centering device (1) for a plate (2) to be used in orthopedic surgery, preferably distal radius surgery, of the present invention comprises the following components or parts:
- A "C" shaped central body including a couple of ends (102);
- A central channel (101) which is on the middle portion of the central body between both ends (102) and is used to place the plate (2) to be used during surgery;
- A fastening channel (103) surrounding the outer edge of the centering device (1) and allowing said device (1) to be secured by the surgeon by means of clamps; and
- A fastening orifice (104) located on the inside portion of the central channel (101) and allowing the device to be attached to the patient's bone (3) by means of a screw that goes through it along with the plate (2)

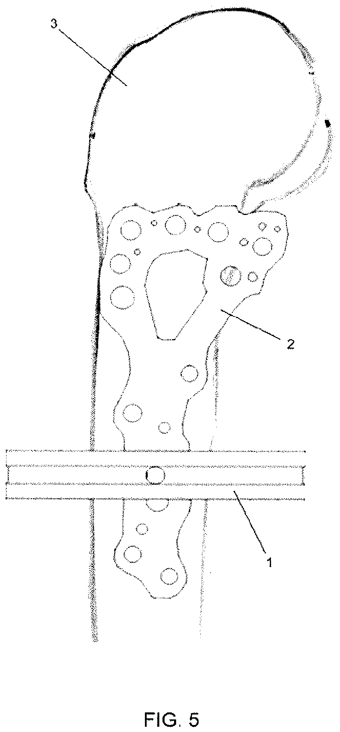

Therefore, the centering device (1) is used to center the plate (2) during orthopedic surgery, special distal radius surgery. In a similar way, the device (1) is used on peri-articular fracture surgery, where device (1) is placed around the bone (3) where the plate (2) is going to be installed to correct or repair the fracture, as shown in figure 5.

Similarly, the central channel (101) has a width corresponding exactly to one of the widths of plate (2), which depends directly on the type of surgery being carried out, in other words, depends on the fractured bone to be mended.

In an embodiment of the present invention, the ends (102) finish on a tip, in other words, the outer edge and the inner edge meet at a tip on each of both ends (102), so as to allow the device (1) to be fitted to the patient in an exact shape and preventing an excess material from the body of the centering device 81), thus reducing the manufacturing costs thereof.

In a preferred embodiment, the centering device (1) is manufactured form a highly elastic polymeric material, which allows it to adapt to the different bone morphologies. In this manner, by being elastic the centering device (1) is wrapped around the bone on the diaphysary portion, guaranteeing that the plate 82) does not lose its orientation while it is being attached to the epiphysiary portion, wherein said attachment can be supported by a clamp that increases pressure on the bone, where the clamp grabs the device (1) by means of channel (103).

## Claims

1. A centering device (1) for a plate (2) to be used in orthopedic surgery, comprising the following components or parts:
• a "C" shaped central body including two opposite ends (102);
• a central channel (101) which is located on a middle portion of the central body between said two ends (102), said central channel (101) being usable to place a plate (2) to be used during surgery; and
• a fastening orifice (104) located on an inside portion of said central channel (101) and allowing the device to be attached to a patient's bone (3) by means of a screw that goes through said fastening orifice (104) along with the plate (2),
**characterized by** further comprising:
a fastening channel (103) surrounding an outer edge of the centering device (1) from one to the other of the two ends (102), said fastening channel (103) allowing the centering device (1) to be secured by a surgeon to the patient's bone (3) by means of a clamp that grabs the centering device (1) by the fastening channel (103) so as to increase pressure on the patient's bone (3).

2. The centering device (1) according to claim 1, **characterized in that** the central channel (101) centers a plate (2) having at least one width, said central channel (101) having a width corresponding exactly to said at least one width of said plate (2), which depends directly on the type of surgery being carried out.

3. The centering device (1) according to claim 1 or 2, **characterized in that** the ends (102) finish on a tip with an outer edge and an inner edge of the central body meeting at said tip on each of both ends (102).

4. The centering device (1) according to any of the previous claims, **characterized in that** the centering device (1) is manufactured form a highly elastic polymeric material, which allows it to adapt to the different bone morphologies.

## Patentansprüche

1. Zentriervorrichtung (1) für eine Platte (2) zur Verwendung in der orthopädischen Chirurgie, umfassend folgende Komponenten oder Teile:
• einen "C"-förmigen Zentralkörper mit zwei gegenüberliegenden Enden (102);
• einen Zentralkanal (101), der sich auf einem mittleren Abschnitt des Zentralkörpers zwischen den beiden Enden (102) befindet, wobei der Zentralkanal (101) verwendbar ist, um eine Platte (2) zu platzieren, die während der Operation verwendet werden soll; und
• eine Befestigungsöffnung (104), die sich auf einem inneren Abschnitt des Zentralkanals (101) befindet und es ermöglicht, die Vorrichtung mit Hilfe einer Schraube, die durch die Befestigungsöffnung (104) zusammen mit der Platte (2) geht, am Knochen (3) eines Patienten zu befestigen,
**dadurch gekennzeichnet, dass** sie ferner folgendes umfasst:
einen Befestigungskanal (103), der eine Außenkante der Zentriervorrichtung (1) von einem der beiden Enden (102) zum anderen umgibt, wobei der Befestigungskanal (103) es ermöglicht, dass die Zentriervorrichtung (1) von einem Chirurgen am Knochen (3) des Patienten mittels einer Klemme befestigt wird, die die Zentriervorrichtung (1) durch den Befestigungskanal (103) erfasst, um den Druck auf den Knochen (3) des Patienten zu erhöhen.

2. Zentriervorrichtung (1) nach Anspruch 1, **dadurch gekennzeichnet, dass** der Zentralkanal (101) eine Platte (2) mit mindestens einer Breite zentriert, wobei der Zentralkanal (101) eine Breite aufweist, die genau der mindestens einen Breite der Platte (2) entspricht, die direkt von der Art der durchzuführenden Operation abhängt.

3. Zentriervorrichtung (1) nach Anspruch 1 oder 2, **dadurch gekennzeichnet, dass** die Enden (102) in einer Spitze enden, wobei eine Außenkante und eine Innenkante des Zentralkörpers an der Spitze an jedem der beiden Enden (102) zusammenkommen.

4. Zentriervorrichtung (1) nach einem der vorhergehenden Ansprüche, **dadurch gekennzeichnet, dass** die Zentriervorrichtung (1) aus einem hochelastischen Polymermaterial hergestellt ist, welches die Anpassung an die verschiedenen Knochenmorphologien ermöglicht.

## Revendications

1. Dispositif de centrage (1) pour une plaque (2) destinée à être utilisée en chirurgie orthopédique, comprenant les composants ou parties suivants:
• un corps central en forme de « C » comportant deux extrémités opposées (102) ;
• un canal central (101) qui est situé sur une partie médiane du corps central entre lesdites deux extrémités (102), ledit canal central (101) étant utilisable pour placer une plaque (2) destinée à être utilisée pendant la chirurgie ; et
• un orifice de serrage (104) situé sur une portion intérieure dudit canal central (101) et permettant au dispositif d'être relié à un os de patient (3) au moyen d'une vis qui passe par ledit orifice de serrage (104) le long de la plaque (2),
**caractérisé en ce qu'**il comprend en outre:
un canal de serrage (103) entourant un bord externe du dispositif de centrage (1) de l'une à l'autre des deux extrémités (102), ledit canal de serrage (103) permettant au dispositif de centrage (1) d'être fixé par un chirurgien à l'os du patient (3) au moyen d'une pince qui accroche le dispositif de centrage (1) par le canal de serrage (103) de sorte à augmenter la pression sur l'os du patient (3).

2. Dispositif de centrage (1) selon la revendication 1, **caractérisé en ce que** le canal central (101) centre une plaque (2) ayant au moins une largeur, ledit canal central (101) ayant une largeur correspondant exactement à ladite au moins une largeur de ladite plaque (2), qui dépend directement du type de chirurgie étant réalisé.

3. Dispositif de centrage (1) selon la revendication 1 ou 2, **caractérisé en ce que** les extrémités (102) terminent sur une pointe avec un bord externe et un bord interne du corps central se joignant à ladite pointe sur chacune des deux extrémités (102).

4. Dispositif de centrage (1) selon l'une quelconque des revendications précédentes, **caractérisé en ce que** le dispositif de centrage (1) est fabriqué à partir d'un matériau polymère élastique, qui lui permet de s'adapter aux différentes morphologies osseuses.
